(19) Europäisches Patentamt
European Patent Office
Office européen des brevets

(11) **EP 2 067 450 A1**

(12) **EUROPEAN PATENT APPLICATION**
published in accordance with Art. 153(4) EPC

(43) Date of publication:
**10.06.2009 Bulletin 2009/24**

(51) Int Cl.:
**A61B 19/00** (2006.01)

(21) Application number: **07740125.5**

(22) Date of filing: **28.03.2007**

(86) International application number:
**PCT/JP2007/056687**

(87) International publication number:
**WO 2008/038427 (03.04.2008 Gazette 2008/14)**

(84) Designated Contracting States:
**AT BE BG CH CY CZ DE DK EE ES FI FR GB GR HU IE IS IT LI LT LU LV MC MT NL PL PT RO SE SI SK TR**
Designated Extension States:
**AL BA HR MK RS**

(30) Priority: **28.09.2006 JP 2006263846**

(71) Applicant: **WASEDA UNIVERSITY**
**Shinjuku-ku Tokyo 169-8050 (JP)**

(72) Inventors:
• **FUJIE, Masakatsu**
**Shinjuku-ku**
**Tokyo 169-8555 (JP)**

• **OKAMOTO, Haruna**
**Shinjuku-ku**
**Tokyo 169-8555 (JP)**
• **OKAMOTO, Jun**
**Shinjuku-ku**
**Tokyo 169-8555 (JP)**

(74) Representative: **Schuster, Thomas**
**Grünecker Kinkeldey Stockmair & Schwanhäusser**
**Anwaltssozietät**
**Leopoldstrasse 4**
**80802 München (DE)**

(54) **SENSING SYSTEM EMPLOYING MEDICAL MANIPULATOR AND PRESSING FORCE MEASURING DEVICE AND ITS PROGRAM**

(57)     Sensing of the pressing force of a balloon against internal body tissues is made possible using the balloon inserted into a gap in the body and pressing the internal body tissues around that gap without inserting a sensor, or the like, separately into the gap. A sensing system comprises an surgical assist manipulator provided to be able to travel by a reaction produced while it pressure-displaces the internal body tissues, a pressure sensor for measuring the pressure of fluid used to operate the surgical assist manipulator, and a pressing force measuring device for determining a pressing force applied to the internal body tissues by the surgical assist manipulator based on the measurement by the pressure sensor. The surgical assist manipulator includes a balloon which can be expanded/contracted by fluid being supplied internally, and the pressing force measuring device determines a function employing the internal pressure when the balloon is pressed as a parameter based on the measurement of fluid amount supplied to the balloon, and determines the pressing force by substituting the internal pressure at pressing measured by the pressure sensor into that function.

FIG.1

## Description

Technical Field

The present invention relates to a sensing system employing a medical manipulator and a pressing force measuring device and its program, and more particularly, to a sensing system employing a medical manipulator and a pressing force measuring device and its program which can use a medical manipulator advancing in a gap in a body in contact with an internal tissue by expanding and contracting a balloon to obtain a pressing force applied to the internal tissue by the balloon.

Background Art

Recent studies have drawn attention to a minimally invasive surgery for reducing the burden of patients without the need to make a large incision, and various surgical assist robot systems for the purpose have been researched and developed. Such a robot system inserts a manipulator accessing an affected part into a body from a hole opened on a body surface and operates the manipulator by remote control. As a manipulator for use in such a system, the present applicants have already proposed a medical manipulator which can autonomously advance in a gap in a body by expansion and contraction of a balloon (see Patent Document 1). The manipulator includes a plurality of bases bendably connected to each other and a balloon which is provided for each base and is expandable and contractible by fluid supplied thereinto. The manipulator expands and contracts the balloon in an arbitrary position as needed to allow an arbitrary balloon to be in contact with an internal tissue therearound and use a reaction force from the internal tissues to autonomously advance the balloon through the gap in the body like a looper. This conventional technique has an advantage in that there is no need to have a mechanism and a power of a motor or the like to advance the manipulator toward the affected part.

Patent Document 1: WO2004/103197

Disclosure of the Invention

Problems to be Solved by the Invention

However, in order to use the manipulator disclosed in the Patent Document 1, since the internal tissues such as brain tissues and organ tissues are pressed (pressure-displaced) by a balloon and a reaction force from that can be used to secure a gap for the manipulator to advance in, the internal tissues need to be prevented from being damaged or contused when the internal tissues are pressure-displaced by the balloon. More specifically, since a damage to a flexible internal tissue or a contusion by an excessive pressure-displace may cause aftereffects after surgical operation, the balloon needs to be controlled such that a pressing force (pressure-displacing force) applied to the internal tissues from the balloon is sensed with reasonable accuracy, and the pressing force acting on the internal tissue should be minimum enough to apply a desired propulsive force to the manipulator depending on the state such as hardness of the internal tissue. Here, the gap in the internal tissue in which the manipulator advances may be extremely narrow and a manipulator having other medical treatment instruments may also advance in the gap. Therefore, it is actually difficult to approach additionally a sensor or the like for measuring the pressure-displacing force to the internal tissue.

In view of the above problems, the present invention has been devised, and an object of the present invention is to provide a sensing system employing a medical manipulator and a pressing force measuring device and its program capable of sensing a pressing force applied to an internal tissue from a balloon without the need to insert a sensor or the like separately in a gap when the manipulator using the balloon advances in the gap in the body.

Means for Solving the Problems

(1) In order to achieve the above object, the present invention provides a configuration in which the present invention is a sensing system employing a medical manipulator which has a balloon inserted in a gap in a living body and expandable and contractible by fluid supplied thereinto, and presses an internal tissue around the gap by expansion of the balloon,
wherein the sensing system comprises: an internal pressure measuring sensor which can measure an internal pressure of the balloon; and a pressing force measuring device which measures a pressing force of the balloon to the internal tissue based on the internal pressure measured by the internal pressure measuring sensor,
wherein the pressing force measuring device comprises: a function determining section which determines a function using, as a parameter, a pressing internal pressure, which is the internal pressure of the balloon when the internal tissue is pressed, based on an amount of fluid supplied into the balloon; and a pressing force operating section which calculates the pressing force by assigning, to the function, the pressing internal pressure measured by the internal pressure measuring sensor.

(2) Here, the configuration is preferably made such that the function determining section preliminarily stores a function for determining a predetermined correction coefficient and constant using the amount of fluid supply as a parameter therein, uses a measured value of the amount of fluid supply to determine the correction coefficient and the constant, and adds the constant to the pressing internal pressure multiplied by the correction coefficient to determine a function for determining the pressing force.

(3) Further, the configuration can also be made such that the pressing force measuring device further comprises a no loading pressure operating section which uses the amount of fluid supply to calculate a no loading internal pressure of the balloon when the balloon is not in contact with the internal tissue, wherein the function determining section determines the function based on the no loading internal pressure calculated by the no loading pressure operating section.

(4) Further, the present invention provides a configuration in which the present invention is a sensing system employing a medical manipulator which has a plurality of balloons bendably connected to each other and expandable and contractible by fluid supplied thereinto, and can adjust a pressing force to an internal tissue due to expansion of the balloon to advance in a gap in a living body using a reaction force from the internal tissue, wherein the sensing system comprises: an internal pressure measuring sensor which can measure an internal pressure of the balloon; and a pressing force measuring device which measures the pressing force based on the internal pressure measured by the internal pressure measuring sensor, wherein the pressing force measuring device comprises: a function determining section which determines a function using, as a parameter, a pressing internal pressure of the balloon when the internal tissue is pressed, based on an amount of fluid supplied into the balloon; and a pressing force operating section which calculates the pressing force by assigning, to the function, the pressing internal pressure measured by the internal pressure measuring sensor.

(5) In the above, the configuration is preferably made such that the medical manipulator comprises a plurality of bases which are bendably connected to each other and to which the individual balloons are attached, wherein the base has a hole which accommodates an open end of the balloon and can close the inside of the balloon, and the open end is connected to a tube passing the fluid.

(6) Further, the present invention provides a configuration in which the present invention is a pressing force measuring device which uses a measured value of an internal pressure of a balloon inserted in a gap in a living body and expandable and contractible by fluid supplied thereinto, to measure a pressing force of the balloon to an internal tissue around the gap, wherein the pressing force measuring device comprise: a function determining section which determines a function using, as a parameter, a pressing internal pressure, which is the internal pressure of the balloon when the internal tissue is pressed, based on a measured value of an amount of fluid supplied into the balloon; and a pressing force operating section which calculates the pressing force by assigning, to the function, the measured value of the internal pressure.

(7) Further, the present invention provides a configuration in which the present invention is a program for causing a pressing force measuring device to execute a process of using a measured value of an internal pressure of a balloon inserted in a gap in a living body and expandable and contractible by fluid supplied thereinto to measure a pressing force of the balloon to an internal tissue around the gap, wherein the program causes the pressing force measuring device to execute a process of determining a function using, as a parameter, a pressing internal pressure, which is the internal pressure of the balloon when the internal tissue is pressed, based on a measured value of an amount of fluid supplied into the balloon; and calculating the pressing force by assigning the measured value of the pressing internal pressure to the function.

It should be noted that in the specification, unless otherwise specified, the term "front" means a leading end of the medical manipulator in a direction of advancing toward a target such as an affected part from outside the body and the term "rear" means the opposite side.
Further, unless otherwise specified, "longitudinal direction" means a direction along the advancing direction of the medical manipulator, and the term "width direction" means a direction orthogonal to the longitudinal direction in a plan view of the medical manipulator. Here, "side view" means a state in which the medical manipulator is viewed in the width direction from outside.
Further, unless otherwise specified, the term "upper" means one end of one of the two directions orthogonal to the advancing direction of the medical manipulator which direction does not correspond to the width direction, and term "lower" means the other end side of the opposite side.

Advantages of the Invention

According to the present invention, the balloon is inserted in a gap in the body and fluid is supplied into the balloon to expand the balloon to press the internal tissue, thereby allowing the balloon to be used as a medical instrument. Further, a pressing force (pressure-displacing force) of the balloon against the internal tissue can be obtained by measuring the amount of fluid supplied into the balloon and the internal pressure of the balloon at that time. Accordingly, the pressing force applied to the internal tissue from the balloon can be sensed without the need to insert a sensor or the like separately in the gap. Even if the gap is narrow, fluid can be supplied into the balloon in consideration of the safety of the living body.

Particularly, according to the configuration (4), the manipulator autonomously advancing in a gap in the body using expansion and contraction of the balloon can have a function as a sensor measuring a pressing force applied to the internal tissue at the advancing, and thus the manipulator using the balloon can sense a pressing force applied from the balloon to the internal tissue without the need to insert a sensor or the like separately in the gap when the manipulator advances in the gap in the body toward an affected part.

Further, the configuration (5) allows the fluid to be reliably supplied without leakage into the balloon from fluid supply means attached to the medical manipulator and provided outside the body, and thus the pressing force can easily obtained from the internal pressure when the balloon is pressed without considering the decrease in pressure inside the balloon due to fluid leakage.

Brief Description of the Drawings

Figure 1 is a schematic configuration view of a sensing system of an surgical assist manipulator in accordance with the present embodiment;
Figure 2 is an enlarged exploded perspective view of the portion A of Figure 1;
Figure 3 is a schematic sectional side view of the surgical assist manipulator;
Figure 4 is a schematic sectional plan view of the surgical assist manipulator;
Figure 5 is an enlarged exploded perspective view of the base;
Figures 6 (A) to (C) are schematic drawings for explaining a procedure for advancing the surgical assist manipulator; and
Figures 7 (A) to (C) are schematic drawings for explaining a procedure for advancing the surgical assist manipulator following Figure 6.

Description of Symbols

10   Sensing system
11   Surgical assist manipulator (medical manipulator)
14   Pressure sensor (internal pressure measuring sensor)
16   Pressing force measuring device
26   Tube
38   Base
39   Balloon
49   Open end
51   No loading pressure operating section
52   Function determining section
53   Pressing force operating section

Best Mode for Carrying Out the Invention

Hereinafter, the embodiments of the present invention will be described with reference to the drawings.

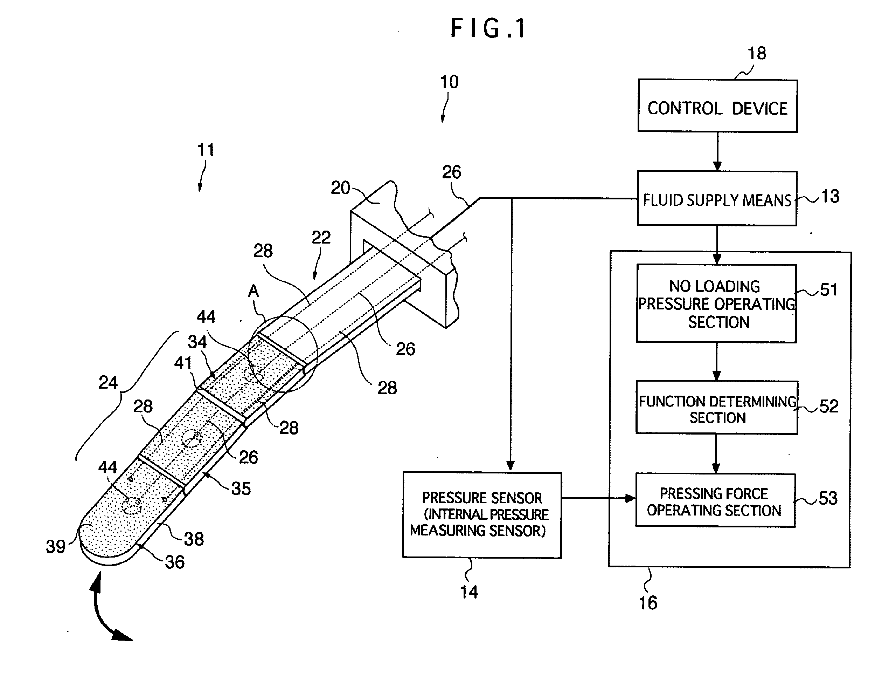

Figure 1 is a schematic configuration view of a sensing system of a medical manipulator in accordance with the present embodiment. With reference to the figure, a sensing system 10 is configured to include: an surgical assist manipulator 11 (medical manipulator) which is provided so as to be able to advance by pressure-displacing, namely, pressing internal tissues and using its reaction force when inserted into a body in surgery; fluid supply means 13 which supplies fluid for operating the surgical assist manipulator 11, into the surgical assist manipulator 11; a pressure sensor 14 which is provided between the surgical assist manipulator 11 and the fluid supply means 13 to measure the pressure of the fluid; a pressing force measuring device 16 which measures the pressing force applied to an internal tissue by the surgical assist manipulator 11 based on the value measured by the pressure sensor 14; and a control device 18 which controls the operation of the surgical assist manipulator 11 by adjusting the amount of fluid supplied to the surgical assist manipulator 11 from the fluid supply means 13.

The surgical assist manipulator 11 is applied to an surgical assist robot and is controlled so as to perform a desired operation in the body by remote control of a doctor and the like. Here, the surgical assist manipulator 11 is an articulated manipulator used mainly for the purpose of expandably opening a gap in the body. The manipulator is used to expandably open a gap or a path from a body surface to an affected part so that other instrument manipulators (not shown) having a treatment instrument such as an endoscope and a scalpel at its front side can easily pass through the path.

The surgical assist manipulator 11 is configured to include: a base end member 22 which is supported in a cantilever state with its rear end side held by the holding body 20 and constitutes a holding portion of the holding body 20; a front end bending member 24 which is provided frontward of the base end member 22 and can be bent in up and down directions with respect to the base end member 22; six tubes 26 (only one tube illustrated in Figure 1) which extend into the front end bending member 24 passing through inside the base end member 22 from the fluid supply means 13 respectively and are housed collectively in substantially center position in the width direction; and two wires 28 and 28 at both sides in the width direction which extend into the front end bending member 24 passing through inside the base end member 22 from the holding body 20 side.

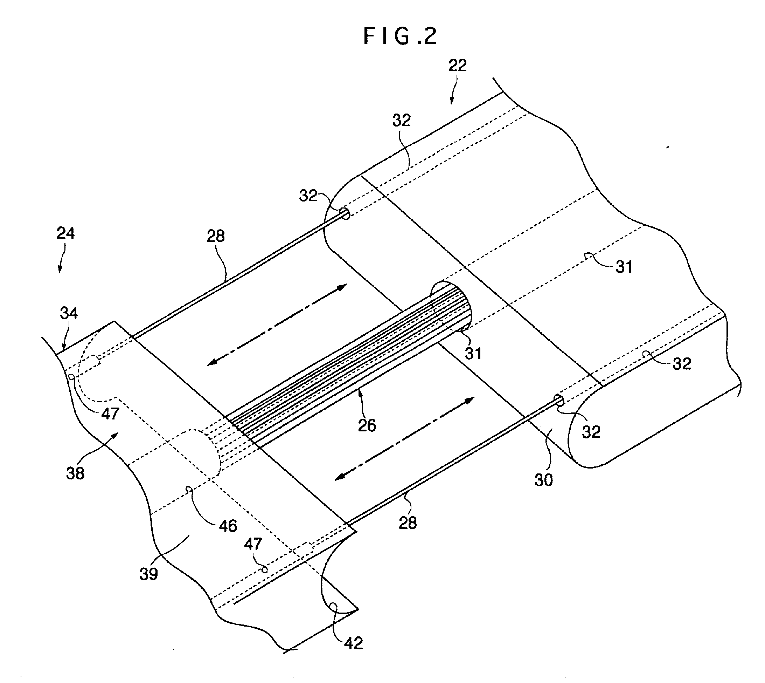

The base end member 22 is formed in a substantially rectangular plate shape in a plan view, and as shown in Figure 2 which is an enlarged exploded perspective view of the portion A in Figure 1, is configured to include: a front end surface 30 whose front end side is formed in a rounded convex curved surface shape; a tube internal space 31 which is formed in substantially the center in the width direction extending in a longitudinal direction and housing the individual tubes 26 collectively; and a wire internal space 32, each of which is formed one by one at both end sides in the width direction extending in a longitudinal direction and housing one of the wires 28 and 28. The individual internal spaces 31 and 32 pass through in the front and rear direction, and the tubes 26 and the wires 28 extending from the holding body 20 side pass through the individual internal spaces 31 and 32 extending to the front end bending member 24 side respectively.

The front end surface 30 is formed in an arc shape in a side view and in a curved surface shape projecting gradually toward substantially the center from both the top and bottom end sides.

As shown in Figure 1, the front end bending member 24 includes: a rear portion forming body 34 continuously connected to the base end member 22 side; a middle forming body 35 continuously connected to the front end side of the rear portion forming body 34; and a front end forming body 36 continuously connected to the front end side of the middle forming body 35.

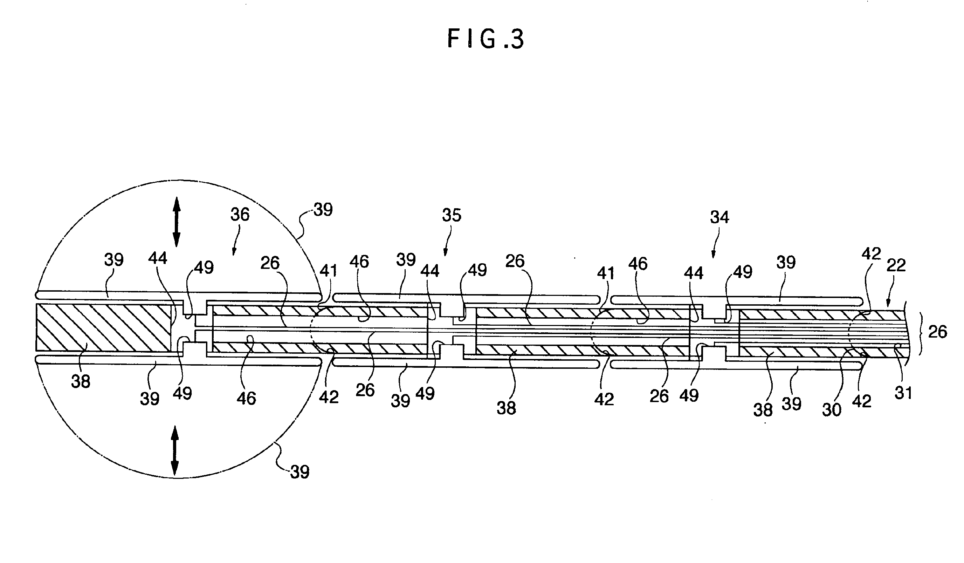

As shown in Figure 3, the forming bodies 34 to 36 include a plate-shaped base 38 and a balloon 39 provided on both the top and bottom surface sides of the base 38 respectively.

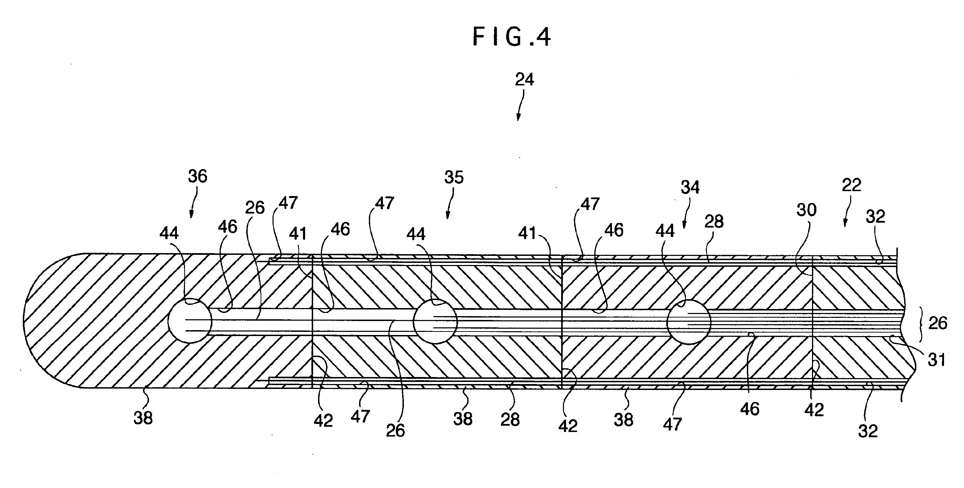

As shown in Figures 3 to 5, the base 38 is configured to include: a front end surface 41 and a rear end surface 42 both of which are formed in a curved surface shape; a hole 44 passing through between both the top and bottom surfaces; a tube internal space 46 which is formed in substantially the center in the width direction thereinside and housing the individual tubes 26 collectively; and a wire internal space 47, each of which is formed one by one on both end sides in the width direction thereinside and housing one of the wires 28 and 28.

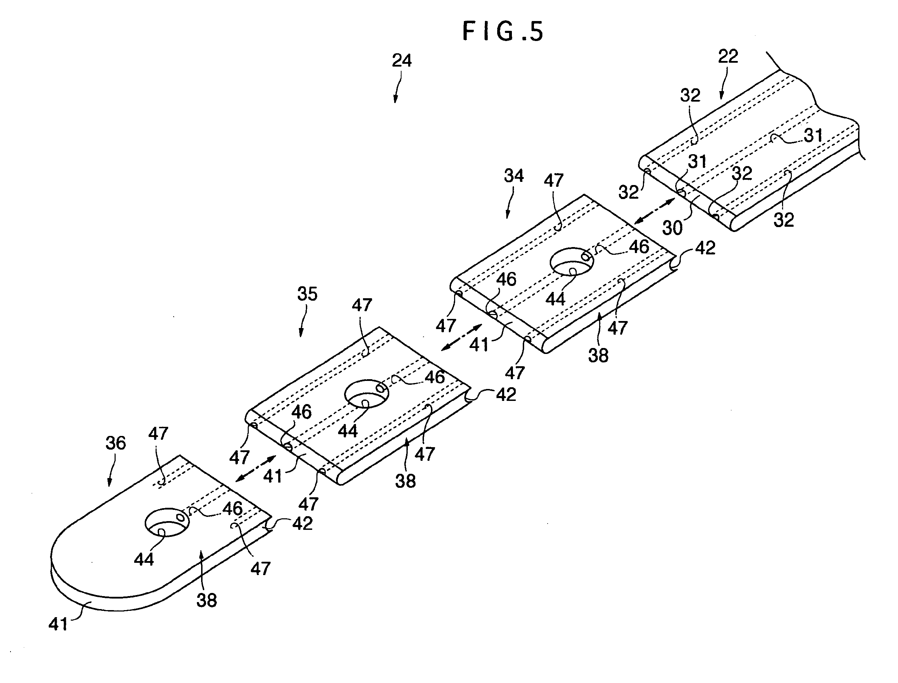

As shown in Figures 3 and 5, the rear end surface 42 of the rear portion forming body 34 is formed in a rounded concave curved surface shape and can be fitted to the front end surface 30 of the base end member 22 substantially without a gap therebetween. More specifically, the rear end surface 42 is formed in an arc shape in a side view gradually concaved inward toward substantially the center upward and downward from both the top and bottom end sides. The front end surface 41 of the rear portion forming body 34 is formed in a rounded convex curved surface shape, that is, a curved surface shape in an arc shape in a side view gradually projecting outward toward substantially the center upward and downward from both the top and bottom end sides.

The rear end surface 42 of the middle forming body 35 is formed in the same curved surface shape as that of the rear end surface 42 of the rear portion forming body 34, and can be fitted to the front end surface 41 of the rear portion forming body 34 substantially without a gap therebetween. The front end surface 41 of the middle forming body 35 is formed in the same curved surface shape as that of the front end surface 41 of the rear portion forming body 34.

The rear end surface 42 of the front end forming body 36 is formed in the same curved surface shape as that of the rear end surface 42 of the middle forming body 35 and can be fitted to the front end surface 41 of the middle forming body 35 substantially without a gap therebetween. In contrast to the front end surfaces 41 and 41 of the rear portion forming body 34 and the middle forming body 35, the front end surface 41 of the front end forming body 36 is formed in a curved surface shape gradually projecting forward to substantially the center from both sides in the width direction.

As shown in Figures 3 to 5, the tube internal spaces 46 and the wire internal spaces 47 and 47 of the individual forming bodies 34 to 36 are communicatively connected to each other. Further, the rear end sides of the spaces 46 and 47 are communicatively connected to the tube internal space 31 and the wire internal spaces 32 and 32 of the base end member 22.

More specifically, the tube internal space 46 of the rear portion forming body 34 extends forward and rearward so as to intersect a hole 44 and to be communicatively connected to the hole 44. Both end sides thereof in forward and rearward directions are opened outward, and the open portion formed on the rear end surface 42 substantially mutually faces the open portion of the tube internal space 31 formed on the front end surface 30 of the base end member 22.

The tube internal space 46 of the middle forming body 35 has the same configuration as the tube internal space 46 of the rear portion forming body 34. The open portion of the tube internal space 46 formed on the rear end surface 42 of the middle forming body 35 substantially mutually faces the open portion of the tube internal space 46 formed on the front end surface 41 of the rear portion forming body 34.

The tube internal space 46 of the front end forming body 36 extends forward into the hole 44 from the rear end surface 42 of the front end forming body 36. The open portion of the tube internal space 46 formed on the rear end surface 42 of the front end forming body 36 substantially mutually faces the open portion of the tube internal space 46 formed on the front end surface 41 of the middle forming body 35.

The wire internal spaces 47 and 47 of the rear portion forming body 34 extend in forward and rearward directions. Both end sides thereof in forward and rearward directions are opened outward, and the open portion formed on the rear end surface 42 substantially mutually faces the open portion of the wire internal spaces 32 and 32 formed on the front end surface 30 of the base end member 22.

The wire internal spaces 47 and 47 of the middle forming body 35 have the same configuration as the wire internal spaces 47 and 47 of the rear portion forming body 34. The open portion of the wire internal spaces 47 and 47 formed on the rear end surface 42 of the middle forming body 35 substantially mutually faces the open portion of the wire internal spaces 47 and 47 formed on the front end surface 41 of the rear portion forming body 34.

The wire internal spaces 47 and 47 of the front end forming body 36 are opened only at the rear end surface 42 side of the front end forming body 36, and extend forward part way to the inside thereof from the open portion. The open portion of the wire internal spaces 47 and 47 substantially mutually faces the open portion of the wire internal spaces 47 and 47 formed on the front end surface 41 of the middle forming body 35.

The total of six balloons 39 are provided one by one on both the top and bottom end sides of the individual forming bodies 34 to 36 so as to supply fluid inside through the tube 26 from the fluid supply means 13 (see Figure 1) and is formed in a bag shape with one end thereof opened by an elastic body such as rubber or the like which can be expanded and contracted by the fluid. More specifically, as shown in Figure 3, the balloon 39 is closed with the open end 49 thereof inserted into the hole 44 so as to prevent fluid inside the balloon 39 from leaking outside. The six tubes 26 extending through inside the tube internal spaces 31 and 46 from the fluid supply means 13 are connected to the open end 49 side of the balloon 39 by one for each balloon 39. Therefore, fluid is supplied independently into the individual balloons 39 from the fluid supply means 13, which allows the balloon 39 to be expanded outward as shown by the one-dot chain line in Figure 3. Here, as described later, when the surgical assist manipulator 11 is inserted into a body, an individual balloon 39 is expanded as needed, so as to cause the balloon 39 to be in contact with an internal tissue therearound. In this expanded state, when the fluid inside the balloon 39 is drawn from the fluid supply means 13 side, the balloon 39 is contracted.

It should be noted that according to the present embodiment, saline is used as the fluid supplied into the balloon 39, but the present invention is not limited to saline, but any other liquid or gas may be applied as long as an unexpected leakage does not cause a problem related to biocompatibility such as contamination in the body or the like and a problem related to expansion and contraction operation and the like.

The tube 26 is formed of flexible and stretchable materials and thus, as described later, can be transformed according to the movement of the base end member 22 and the individual forming bodies 34 to 36 even when each of them is bent and displaced upward and downward relatively to each other.

One end of the wire 28 is fixed to the inside of the front end forming body 36, and the other end thereof is linked to a wire traction device (not shown) through the wire internal spaces 32 and 47. Driving the wire traction device allows the tension of the wire 28 to be arbitrarily changed so that the wire 28 can be changed between a tense state and a relaxed state.

As described above, the surgical assist manipulator 11 allows the base end member 22 and the individual forming bodies 34 to 36 to be connected to each other through the tubes 26 and the wires 28. When the wire 28 is in a relaxed state, and an external force is applied to the base end member 22 and the individual forming bodies 34 to 36, the rear end surface 42 of one member in the link regions (joint regions) can slide along the front end surfaces 30 and 41 of the other member so as to be bendable upward and downward with each other. In order to prevent the base end member 22 and the individual forming bodies 34 to 36 from being bent and displaced upward and downward, all that is needed is to use the wire traction device to strongly pull the wire 28 so as to cause the individual link regions to be in close contact with each other. By doing so, the front end bending member 24 can be locked so as not to be bent and displaced.

Various instruments and devices such as an injection instrument such as a syringe and a liquid filling device using a pump may be applied to the fluid supply means 13 as long as fluid can be supplied and discharged to and from each of the six tubes 26 independently. The configuration of the fluid supply means 13 is not within the scope of the present invention, and thus the detailed description is omitted.

The surgical assist manipulator 11 uses the above configuration to operate as follows.

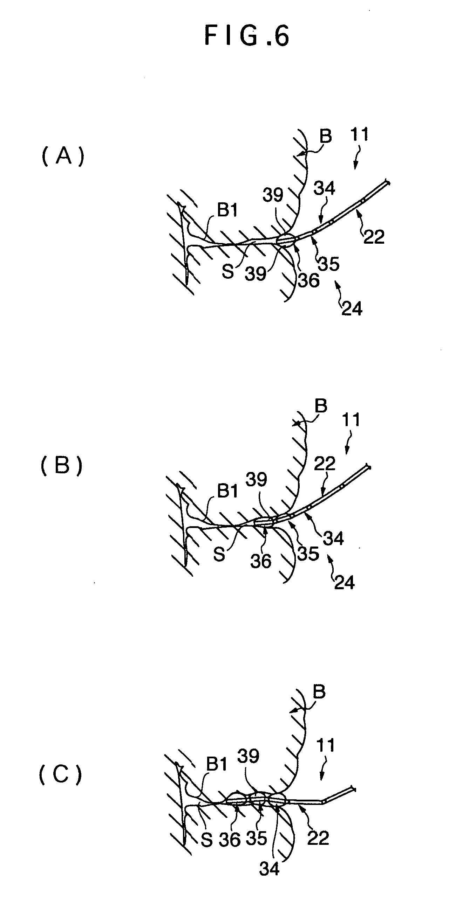

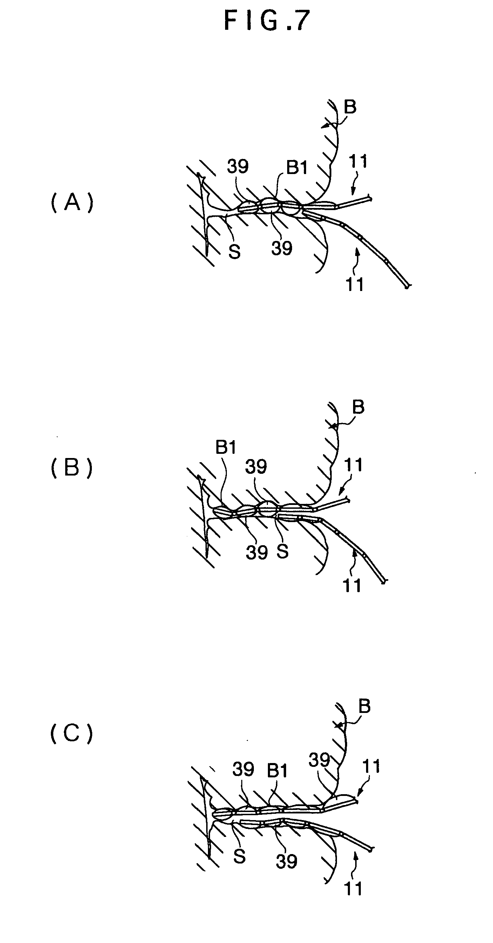

For example, when the surgical assist manipulator 11 is inserted into a brain, as shown in Figure 6 (A), the front end side thereof is set in the vicinity of an inlet of a gap S in the brain B. Then, fluid is supplied into the balloon 39 from the fluid supply means 13 to expand the balloon 39 from the front end side (see Figures (A) to (C)). At this time, the balloon 39 having a predetermined elasticity gently presses the brain tissue B1 around the gap S. Then, the control device 18 (see Figure 1) can be used to independently control the amount of fluid to be supplied to the individual balloons 39 and 39 on the mutually facing both the top and bottom sides to adjust the pressing force to the brain tissue B1 for each balloon 39. In this manner, the pressing force to the brain tissue B1 can be adjusted according to the shape of the gap S. The surgical assist manipulator 11 uses a reaction force from the brain tissue B1 to cause the individual forming bodies 34 to 36 of the front end bending member 24 to be bent and displaced upward and downward direction following the shape of the gap S. The surgical assist manipulator 11 thus advances toward the affected part while thrusting its way through the gap S like a looper. When the front end side of the surgical assist manipulator 11 reaches the affected part, the wires 28 and 28 (see Figure 1) are pulled to lock the surgical assist manipulator 11 so as to prevent the front end bending member 24 from being displaced relatively. Then, as shown in Figure 7, another surgical assist manipulator 11 is inserted into the gap S in a manner similar to that previously described. The balloon 39 is expanded to expandably open the gap S to secure a passage through which another succeeding instrument manipulator (not shown) can be inserted.

At this time, the pressure sensor 14 and the pressing force measuring device 16 are used to measure the pressing force of the balloon 39 to the brain tissue B1. Then, the control device 18 uses the pressing force to control the fluid supply means 13.

The pressure sensors 14 are provided, one for each tube 26, at the rear end side thereof and can measure a fluid pressure inside the individual tube 26 corresponding to the internal pressure of the individual balloon 39. Therefore, the pressure sensor 14 constitutes an internal pressure measuring sensor which measures the internal pressure of the balloon 39.

The pressing force measuring device 16 is configured of a predetermined computer and a program for causing the computer to function as follows is installed. The program is executed to perform a process of obtaining a pressing force of an individual balloon 39 when the individual balloon 39 is in contact with an internal tissue in consideration of the material quality, size, transformation degree and the like of the balloon 39.

More specifically, as shown in Figure 1, the pressing force measuring device 16 is configured to include: a no loading pressure operating section 51 which uses the amount of fluid supplied into a balloon 39 to determine a no loading internal pressure of the balloon 39 when the balloon 39 is not in contact with an internal tissue; a function determining section 52 which uses the no loading internal pressure determined by the no loading pressure operating section 51 to determine a function using, as a parameter, the pressing internal pressure of the balloon 39 when the balloon 39 presses the internal tissue; and a pressing force operating section 53 which assigns, to the parameter of the function, the pressing internal pressure measured by the pressure sensor 14 to calculate the pressing force.

The no loading pressure operating section 51 uses the amount of fluid supplied into the balloon 39 to determine the no loading internal pressure of the balloon 39, assuming that the balloon 39 is not in contact with the internal tissue and thus, in a no loading state.

More specifically, the following expression (1) is determined according to the material quality, size, and the like of the balloon 39 and is preliminarily stored in the no loading pressure operating section 51. The no loading internal pressure is calculated by the following expression (1).

$$P_1 = aV^2 + b \qquad (1)$$

where

P$_1$: Internal pressure at no loading,
V: Volume of the balloon 39 in no loading state = amount of fluid supply,
a: Constant uniquely determined by the material quality, size, and the like of the balloon, and
b: Internal pressure of the balloon 39 in no loading state assuming that the volume of the balloon 39 is 0.

As described above, the amount of fluid to be supplied to the balloon 39 is equal to the volume V of the balloon in no loading state. Accordingly, the fluid supply means 13 is used to detect the amount of fluid supply and the volume V corresponding to the amount of fluid supply is assigned to the above expression (1) to obtain the no loading internal pressure P$_1$.

The function determining section 52 determines the following expression (2) indicating the relation between the pressing internal pressure of the balloon 39 when the balloon 39 is actually pressing the internal tissue and the pressing force applied to the internal tissue.

$$P_2 = kP_3 + P_1 \qquad (2)$$

where

P$_2$: Pressing internal pressure measured by the pressure sensor 14,
P$_3$: Pressing force to the internal tissue by the balloon 39, and
k: Correction coefficient uniquely determined according to the no loading internal pressure P$_1$.

That is, the correction coefficient k is obtained by the following function using the no loading internal pressure P$_1$ as a parameter.

$$k = f(P_1) \qquad (3)$$

The function (3) is preliminarily stored in the function determining section 52 based on an experiment described later.

Therefore, the no loading internal pressure $P_1$ obtained by the above expression (1) is assigned to the above expression (3) to obtain the correction coefficient k. Then, the function (2) differing according to the no loading pressure $P_1$ is determined.

Here, the function in the above expression (3) is set such that the correction coefficient k decreases with an increase in no loading internal pressure $P_1$, depending on the quality of the balloon 39.

The pressing force operating section 53 uses the above expression (2) determined by the function determining section 52 to determine the pressing force $P_3$. More specifically, the pressing internal pressure $P_2$ measured by the pressure sensor 14 is assigned to the above expression (2) to obtain the pressing force $P_3$ applied to the internal tissue by the balloon 39.

The calculation procedure described above has been devised based on the experiments preliminarily performed by the present inventors. Here, for the experiments, an experimental device is created which can immerse a balloon 39 into a water bath (not shown) and supply fluid into the balloon 39 from outside. More specifically, an assumption is made that water around the balloon 39 is an internal tissue, a water pressure in the water bath corresponds to the pressing force, an amount of fluid supplied into the balloon 39 corresponds to the volume of the balloon 39 in the no loading state, and an internal pressure of the balloon 39 corresponds to the pressing internal pressure. First, each of the water pressure, the amount of fluid supplied and the internal pressure of the balloon 39 is measured by changing one of the water pressure, the amount of fluid supplied and the internal pressure of the balloon 39 to determine their relation. Then, the relation is used to derive the above described calculation procedure.

The control device 18 is configured of a predetermined computer and the fluid supply means 13 is controlled so as to be able to adjust the amount of fluid to be supplied into the individual balloon 39 according to the pressing force of the individual balloon 39 to the internal tissue which is measured by the pressing force measuring device 16. More specifically, when the individual balloon 39 presses the internal tissue, the control device 18 controls the fluid supply means 13 so as to ensure an enough reaction force for the surgical assist manipulator 11 to advance, and when the pressing force of the balloon 39 to the internal tissue exceeds a predetermined threshold, the control device 18 controls the fluid supply means 13 so as to stop supplying fluid into the corresponding balloon 39 or discharge the fluid inside the balloon 39 to outside.

According to the present embodiment, the balloons 39 are expanded and contracted to advance the surgical assist manipulator 11 toward the affected part. Therefore, the balloon 39 functions not only as a sort of actuator for operating the surgical assist manipulator, but also as a sensor capable of measuring the pressing force to the internal tissue based on the pressing internal pressure. Accordingly, only the surgical assist manipulator 11 is enough to control the operation so as to prevent the internal tissue from being damaged or contused without the need to provide a special sensor or the like separately.

It should be noted that as the pressing force measuring device 16, a variation of the embodiment is used to obtain the pressing force $P_3$ without using the no loading pressure operating section 51.

More specifically, the function determining section 52 of the present variation uses the following expression (4) indicating the relation between the pressing internal pressure $P_2$ of the balloon 39 when the balloon is actually pressing the internal tissue and the pressing force $P_3$ to the internal tissue.

$$P_3 = \alpha P_2 + \beta \qquad (4)$$

where,
$\alpha$ is a correction coefficient which is uniquely determined according to the volume V of the balloon 39 in the no loading state, namely, the measured amount of fluid supply, and the correction coefficient $\alpha$ is obtained by the following function (5) using the volume V as a parameter.

$$\alpha = f_{\alpha}(V) \qquad (5)$$

where,
$\beta$ is a constant which is uniquely determined according to the volume V, and the constant $\beta$ is obtained by the following function (6) different from the expression (5) using the volume V as a parameter.

$$\beta = f_{\beta}(V) \qquad (6)$$

The functions (5) and (6) are preliminarily determined based on the above described experiments, and are stored in the function determining section 52.

Therefore, the amount of fluid (volume V) supplied by the fluid supply means 13 is assigned to the above expression (5) and (6) to determine the correction coefficient $\alpha$ and the constant $\beta$ and then determine the function (4) differing according to the amount of fluid supply.

Here, the above functions (5) and (6) are set such that, depending on the quality of the balloon 39, $\alpha$ increases linearly with an increase in volume V, $\beta$ decreases in a curved line with an increase in volume V, and the degree of decrease increases gradually with an increase in volume V.

According to the pressing force operating section 53 in accordance with the present variation, the pressing internal pressure $P_2$ measured by the pressure sensor 14 is assigned to the above expression (4) determined by the function determining section 52 to obtain the pressing force $P_3$. It should be noted that the pressing force calculation procedure in accordance with present variation is also devised based on the experiments performed by the present inventors.

Therefore, according to the present variation, unlike the above embodiments, there is no need to obtain the no loading internal pressure $P_1$, and thus the pressing force $P_3$ of balloon 39 to the internal tissue can be obtained more easily.

It should be noted that the three-joint type front end bending member 24 is illustrated and described as the surgical assist manipulator 11, the number of joints, namely, the configuration of the individual forming bodies 34 to 36 of the front end bending member 24 is not limited to the above described structure as long as the front end bending member 24 is bendably connected to the base end member 22.

Further, the surgical assist manipulator 11 is not limited to the structure of the above embodiments and the variation thereof, but various configurations may be used as long as the balloon 39 or a similar elastic body is used to enable the same operation as described above. For example, the present invention may be applied to the manipulator configuring one balloon 39 or a plurality of balloons 39 and the tube 26 connected to the balloon 39 without using the base 38.

Further, according to the above embodiments and the variation thereof, as the surgical assist manipulator 11 to which the present invention is applied, a description is given to the manipulator having the purpose of expandably opening the gap in the body, the present invention is not limited to the manipulator for this application and the present invention may be applied to other instrument manipulators having a treatment instrument such as an endoscope and a scalpel at its front side. Still further, the present invention may be applied not to an autonomously advancing manipulator, but, for example, to a medical instrument such as a brain retractor which expandably opens the gap in the brain tissue without autonomously advancing. Therefore, the term "medical manipulator" in the present specification and Claims is used as a concept including various medical instruments.

Still further, the configuration of the individual sections of the devices in accordance with the present invention is not limited to the illustrated configuration, but various modifications may be made to the shape, structure, number of elements, and the like as long as the configuration exerts substantially similar effects.

**Claims**

1. A sensing system employing a medical manipulator which has a balloon inserted in a gap in a living body and expandable and contractible by fluid supplied thereinto, and presses an internal tissue around the gap by expansion of the balloon, the sensing system comprising:

an internal pressure measuring sensor which can measure an internal pressure of the balloon; and a pressing force measuring device which measures a pressing force of the balloon to the internal tissue based on the internal pressure measured by the internal pressure measuring sensor,
wherein the pressing force measuring device comprises: a function determining section which determines a function using, as a parameter, a pressing internal pressure, which is the internal pressure of the balloon when the internal tissue is pressed, based on an amount of fluid supplied into the balloon; and a pressing force operating section which calculates the pressing force by assigning, to the function, the pressing internal pressure measured by the internal pressure measuring sensor.

2. The sensing system employing the medical manipulator according to claim 1, the function determining section preliminarily stores a function for determining a predetermined correction coefficient and constant using the amount of fluid supply as a parameter therein, uses a measured value of the amount of fluid supply to determine the correction coefficient and the constant, and adds the constant to the pressing internal pressure multiplied by the correction coefficient to determine a function for determining the pressing force.

3. The sensing system employing the medical manipulator according to claim 1, the pressing force measuring device further comprises a no loading pressure operating section which uses the amount of fluid supply to calculate a no loading internal pressure of the balloon when the balloon is not in contact with the internal tissue,
wherein the function determining section determines the function based on the no loading internal pressure calculated by the no loading pressure operating section.

4. A sensing system employing a medical manipulator which has a plurality of balloons bendably connected to each other and expandable and contractible by fluid supplied thereinto, and can adjust a pressing force to an internal tissue due to expansion of the balloon to advance in a gap in a living body using a reaction force from the internal tissue, the sensing system comprising:

an internal pressure measuring sensor which can measure an internal pressure of the balloon; and a pressing force measuring device which measures the pressing force based on the internal pressure measured by the internal pressure measuring sensor,

wherein the pressing force measuring device comprises: a function determining section which determines a function using, as a parameter, a pressing internal pressure of the balloon when the internal tissue is pressed, based on an amount of fluid supplied into the balloon; and a pressing force operating section which calculates the pressing force by assigning, to the function, the pressing internal pressure measured by the internal pressure measuring sensor.

5. The sensing system employing the medical manipulator according to any of claims 1 to 4, the medical manipulator comprises a plurality of bases which are bendably connected to each other and to which the individual balloons are attached,
wherein the base has a hole which accommodates an open end of the balloon and can close the inside of the balloon, and the open end is connected to a tube passing the fluid.

6. A pressing force measuring device which uses a measured value of an internal pressure of a balloon inserted in a gap in a living body and expandable and contractible by fluid supplied thereinto, to measure a pressing force of the balloon to an internal tissue around the gap, the pressing force measuring device comprising:

a function determining section which determines a function using, as a parameter, a pressing internal pressure, which is the internal pressure of the balloon when the internal tissue is pressed, based on a measured value of an amount of fluid supplied into the balloon; and a pressing force operating section which calculates the pressing force by assigning, to the function, the measured value of the internal pressure.

7. A program of a pressing force measuring device, the program for causing the pressing force measuring device to execute a process of using a measured value of an internal pressure of a balloon inserted in a gap in a living body and expandable and contractible by fluid supplied thereinto to measure a pressing force of the balloon to an internal tissue around the gap,
wherein the program causes the pressing force measuring device to execute a process of determining a function using, as a parameter, a pressing internal pressure, which is the internal pressure of the balloon when the internal tissue is pressed, based on a measured value of an amount of fluid supplied into the balloon; and calculating the pressing force by assigning the measured value of the pressing internal pressure to the function.

# FIG.1

CONTROL DEVICE — 18

FLUID SUPPLY MEANS — 13

NO LOADING PRESSURE OPERATING SECTION — 51

FUNCTION DETERMINING SECTION — 52

PRESSING FORCE OPERATING SECTION — 53

PRESSURE SENSOR (INTERNAL PRESSURE MEASURING SENSOR) — 14

EP 2 067 450 A1

F I G . 2

EP 2 067 450 A1

FIG.3

FIG.4

EP 2 067 450 A1

FIG.5

# FIG.6

(A)

(B)

(C)

# F I G .7

(A)

(B)

(C)

**INTERNATIONAL SEARCH REPORT**

| International application No. |
| --- |
| PCT/JP2007/056687 |

A. CLASSIFICATION OF SUBJECT MATTER
*A61B19/00*(2006.01)i

According to International Patent Classification (IPC) or to both national classification and IPC

B. FIELDS SEARCHED

Minimum documentation searched (classification system followed by classification symbols)
A61B19/00

Documentation searched other than minimum documentation to the extent that such documents are included in the fields searched

| Jitsuyo Shinan Koho | 1922-1996 | Jitsuyo Shinan Toroku Koho | 1996-2007 |
| Kokai Jitsuyo Shinan Koho | 1971-2007 | Toroku Jitsuyo Shinan Koho | 1994-2007 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
| --- | --- | --- |
| A | WO 2004/103197 A1 (Waseda University), 02 December, 2004 (02.12.04), Par. No. [0044] & US 2006/217686 A1 | 1-7 |
| A | JP 2003-527918 A (Wilson-Cook Medical Inc.), 24 September, 2003 (24.09.03), Full text; all drawings & WO 2001/072221 A1 | 1-7 |
| A | JP 10-262935 A (Seiko Epson Corp.), 06 October, 1998 (06.10.98), Full text; all drawings (Family: none) | 1-7 |

☐ Further documents are listed in the continuation of Box C.　　☐ See patent family annex.

| * | Special categories of cited documents: |
| --- | --- |
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
| --- | --- |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search 04 June, 2007 (04.06.07) | Date of mailing of the international search report 12 June, 2007 (12.06.07) |
| --- | --- |
| Name and mailing address of the ISA/ Japanese Patent Office | Authorized officer |
| Facsimile No. | Telephone No. |

Form PCT/ISA/210 (second sheet) (April 2005)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2004103197 A